# EUROPEAN PATENT SPECIFICATION

(11) **EP 4 051 097 B1**
(45) Date of publication and mention of the grant of the patent: **25.03.2026**
(21) Application number: 20883006.7
(22) Date of filing: 29.10.2020
(51) Int. Cl.: A61B 5/00, A61C 17/22, A61C 19/04, A61C 19/06, A61K 8/02, A61K 8/19, A61Q 11/00, A61C 17/34

(54) **AUTOMATED AND PRECISE DEVICE FOR DENTAL PLAQUE DETECTION, MONITORING AND REMOVAL**
AUTOMATISIERTE UND PRÄZISE VORRICHTUNG ZUR DETEKTION, ÜBERWACHUNG UND ENTFERNUNG VON ZAHNBELAG
DISPOSITIF AUTOMATISÉ ET PRÉCIS POUR LA DÉTECTION, LA SURVEILLANCE ET LE RETRAIT DE PLAQUE DENTAIRE

(30) Priority: 29.10.2019 US 201962927414 P
(43) Date of publication of application: 07.09.2022
(73) Proprietor: The Trustees of the University of Pennsylvania, Philadelphia, PA 19104 (US)
(72) Inventor: KOO, Hyun, Philadelphia, PA 19147 (US); STEAGER, Edward, Media, PA 19063 (US); HUNTER, Elizabeth, Philadelphia, PA 19146 (US); BABEER, Alaa, Philadelphia, PA 19104 (US)
(74) Representative: Barker Brettell LLP
(86) International application number: PCT/US2020/057923
(87) International publication number: WO 2021/087088

(56) References cited:
- WO-A1-2018/160986
- WO-A1-2020/112601
- US-A1- 2008 255 498
- US-A1- 2008 255 498
- US-A1- 2010 151 407
- US-A1- 2017 197 070
- US-A1- 2017 197 071
- US-A1- 2018 028 417
- US-A1- 2019 282 348
- US-B2- 8 133 054
- US-B2- 9 968 335
- HWANG GEELSU ET AL: "Catalytic antimicrobial robots for biofilm eradication", SCIENCE ROBOTICS, vol. 4, no. 29, 10 April 2019 (2019-04-10), XP055931999, DOI: 10.1126/scirobotics.aaw2388
- HWANG GEELSU; PAULA AMAURI J; HUNTER ELIZABETH E; LIU YUAN; BABEER ALAA; KARABUCAK BEKIR; STEBE KATHLEEN; KUMAR VIJAY; STEAGER EDW: "Catalytic antimicrobial robots for biofilm eradication", SCIENCE ROBOTS, vol. 4, no. 29, 24 April 2019 (2019-04-24), US , pages 1 - 13, XP009536701, ISSN: 2470-9476, DOI: 10.1126/scirobotics.aaw2388
- GAO ET AL.: "Nanocatalysts promote Streptococcus mutans biofilm matrix degradation and enhance bacterial killing to suppress dental caries in vivo", BIOMATERIALS, vol. 101, 1 September 2016 (2016-09-01), AMSTERDAM, NL , pages 272 - 284, XP055932706, ISSN: 0142-9612, DOI: 10.1016/j.biomaterials.2016.05.051

## Description

### BACKGROUND

Maintaining teeth and gums in healthy condition requires regular self-administered oral care such as teeth brushing, dental flossing and antimicrobial oral rinse. The American Dental Association recommends brushing teeth twice a day for both, adults and children. However, teeth brushing is often performed below recommended standards, e.g., fewer than suggested number of times a day or less than a recommended time interval. Reasons for not performing teeth brushing to recommended standards can include, for example, time and effort required to satisfactorily clean all teeth in the top and bottom jaw, user boredom during the activity, manual dexterity and lack of interest in performing a chore-like activity. Furthermore, optimal plaque removal requires multiple steps such as flossing (between the teeth) and mouth rising to chemically kill bacteria.

Insufficient dental care can lead to the formation of biofilms. Biofilms are structured communities of bacterial cells surrounded by a matrix of extracellular polymeric substances, such as exopolysaccharides (EPS), which can act as a barrier to provide protection against antimicrobials, cohesion to the biofilm structure and mechanical stability for firm adhesion to the surface. Biofilms formed on teeth are associated with dental caries (tooth decay), gingivitis and periodontal diseases.

Certain techniques for combating biofilms on teeth are largely inadequate and cumbersome because they fail to simultaneously kill and physically remove bacteria, while requiring dexterity for manual biofilm removal such as tooth-brushing and dental flossing. Certain antimicrobial agents, such as oral antiseptics and rinses, are incapable of breaking down the biofilm matrix and have limited killing effects against bacteria embedded inside the protected biofilm structure, and the biofilm retains the ability to rapidly reestablish itself if biofilm debris and bacteria are not removed. While certain devices and methods are available to mechanically remove plaque both at the dental office (scraping) and at home (toothbrush, dental flossing and electric brushes), they require manual dexterity.

US2008255498A1 discloses compositions that are photosensitizers and/or sonosensitizers and devices and methods for manufacture, application, and activation of those compositions to kill microorganisms and/or bleach colored compounds.

The techniques discussed above pose challenges to both to consumers who are at risk of plaque accumulation and oral diseases due to lack of compliance, and elderly and patients with physical and cognitive disabilities who may have poor manual dexterity.

These challenges are exasperated by a lack of available techniques for real-time detection and monitoring plaque accumulation or oral health conditions associated with plaque-biofilms.

Accordingly, there exists a need for a device that could simultaneously achieve dental plaque detection, monitoring and removal in an automated manner with high precision.

### SUMMARY

The present invention pertains to an oral care device as defined in the appended claims 1 to 5.

Devices and methods for removing dental biofilms from teeth and eradicating bacteria within such dental biofilms are presented herein.

In certain embodiments, the present disclosure provides oral care devices. An example device includes a flexible mouthpiece having an upper channel configured to fit over upper teeth and a lower channel configured to fit over lower teeth of a user. In certain embodiments, the mouthpiece includes one or more magnetic elements and a vibrating motor. In certain embodiments, the one or more magnetic elements include a permanent magnet and/or electromagnet, adapted to apply a magnetic field to the biofilm to actuate the iron oxide nanoparticles to assemble into antimicrobial robots or autonomous magnetic bristles or robots.

In certain embodiments, the oral care device of the present disclosure further includes a photodetector or RGB sensor. In certain embodiments, the photodetector is coupled to a light-emitting diode.

In certain embodiments, the upper channel and the lower channel of the mouthpiece of the present disclosure contain a suspension including iron oxide nanoparticles, wherein the upper channel and the lower channel of the mouthpiece are configured to provide contact between the suspension and surfaces of upper teeth, lower teeth, between the teeth and gums of the user.

In certain embodiments, the upper channel and the lower channel of the mouthpiece contain a suspension including iron oxide nanoparticles and a component selected from the group consisting of hydrogen peroxide, carbamide peroxide, enzymes, antimicrobial compounds, fluoride ion sources, abrasive compounds, flavonoids, terpenoids, polyphenols, proanthocyanidins, tannins, coumarin, surfactants, detergents, glycerol, rose bengal, perborate, meta-periodate, sorbitol, xylitol, 1-deoxynojirimycin, and combinations thereof. In certain embodiments, the suspension includes hydrogen peroxide. In certain embodiments, the suspension includes one or more enzymes. In certain embodiments, the one or more enzymes is mutanase, dextranase, or combinations thereof.

In certain embodiments, the suspension includes hydrogen peroxide and a peroxidase sensitive dye. In certain embodiments, the peroxidase sensitive dye is 3,3',5,5'-tetramethylbenzidine (TMB). Other dyes include: 2,2'-azino-bis(3-ethylbenzothiazoline-6-sulphonicacid (ABTS), o-phenylenediamine (OPD), 3, 3'-Diaminobenzidine (DAB), Pyrogallol, 4-amino-2,3-dimethyl-1-phenyl-3-pyrazolinone (4-Aminoantipyrine), 5-aminosalicylic acid (5-AS), 3-methyl-2-benzothiazolinone (MBTH) as well as fluorescent dyes including 10-Acetyl-3,7-dihydroxyphenoxazine, Terephthalic acid, Homovanillic acid, 2-[6-(4-aminophenoxy)-3-oxo-3H-xanthen-9-yl]-benzoic acid, (2-[6-(4'-hydroxy)phenoxy-3H-xanthene-3-on-9-yl]benzoic acid, 2',7'-Dichlorofluorescein diacetate, 2,7-Dichlorodihydrofluorescein diacetate, Coumarin Boronic Acid, Coumarin Boronic Acid pinacolate ester, Dihydrorhodamine 123, Lucigenin, Dihydroethidium.

In certain embodiments, the present disclosure provides methods for removing dental biofilm. An example method includes inserting the oral care device of the present disclosure into a mouth of the user, administering a suspension including iron oxide nanoparticles to the upper channel and the lower channel of the mouthpiece, actuating the iron oxide nanoparticles with the one or more magnetic elements for assembly into antimicrobial robots or autonomous magnetic bristles or robots suitable for removal of dental biofilm, and applying the magnetic field to move the antimicrobial robots or autonomous magnetic bristles or robots along the surfaces of upper teeth, lower teeth, including between the teeth, and gums of the user to mechanically remove dental biofilm.

In certain embodiments, the suspension additionally includes a component selected from hydrogen peroxide, carbamide peroxide, enzymes, antimicrobial compounds, surfactants, detergents, fluoride ion sources, abrasive compounds, glycerol, flavonoids, terpenoids, polyphenols, proanthocyanidins, tannins, coumarin, rose bengal, perborate, meta-periodate, sorbitol, xylitol, 1-deoxynojirimycin and combinations thereof.

In certain embodiments, the method for removing dental biofilm includes inserting the oral care device of the present disclosure into a mouth of the user, administering a suspension including iron oxide nanoparticles and hydrogen peroxide to the upper channel and the lower channel. In certain embodiments, the suspension includes from about 0.1% w/w to about 10% w/w, from about 0.5% w/w to about 7.5% w/w, or from about 1% w/w to about 5% w/w of hydrogen peroxide based on total weight of the suspension. In certain embodiments, the suspension can include at least about 0.1% w/w, at least about 0.5% w/w, at least about 1.0% w/w, at least about 2.0% w/w/, at least about 3.0% w/w, at least about 4.0% w/w, at least about 5.0% w/w, at least about 6.0% w/w, at least about 7.0% w/w, at least about 8.0% w/w, at least about 9.0% w/w, or at least about 10% w/w, based on total weight of the suspension. In certain embodiments, the suspension can include less than about 0.5% w/w, less than about 1.0% w/w, less than about 2.0% w/w/, less than about 3.0% w/w, less than about 4.0% w/w, less than about 5.0% w/w, less than about 6.0% w/w, less than about 7.0% w/w, less than about 8.0% w/w, less than about 9.0% w/w, or less than about 10% w/w, based on total weight of the suspension. The iron oxide nanoparticles activate hydrogen peroxide to produce bioactive radicals capable of degrading dental biofilm and eradicating bacteria within dental biofilm. The method further includes actuating the iron oxide nanoparticles with the one or more magnetic elements for assembly into antimicrobial robots or autonomous magnetic bristles or robots suitable for removal of dental biofilm and applying the magnetic field to move the antimicrobial robots or autonomous magnetic bristles or robots along the surfaces of upper teeth, lower teeth, between teeth, and gums of the user to mechanically remove dental biofilm. In certain embodiments, the suspension includes from about 500 micrograms to about 5000 micrograms, from about 750 micrograms to about 4750 micrograms, from about 1000 micrograms to about 4500 micrograms, from about 1250 micrograms to about 4250 micrograms, from about 1500 micrograms to about 4000 micrograms, from about 1750 micrograms to about 3750 micrograms, or from about 2000 micrograms to about 3500 micrograms of iron oxide nanoparticles per milliliter of 50% glycerol in water, or water, or aqueous buffer. In certain embodiments, the suspension includes less than 5000 micrograms, less than 4500 micrograms, less than 4000 micrograms, less than 3500 micrograms, less than 3000 micrograms, less than 2500 micrograms, less than 2000 micrograms, less than 1500 micrograms, or less than 1000 micrograms of iron oxide nanoparticles per milliliter of 50% glycerol in water, or water. In certain embodiments, the suspension includes at least 500 micrograms, at least 1000 micrograms, at least 1500 micrograms, at least 2000 micrograms, at least 2500 micrograms, at least 3000 micrograms, at least 3500 micrograms, at least 4000 micrograms, or at least 4500 micrograms of iron oxide nanoparticles per milliliter of 50% glycerol in water, or water, or aqueous buffer. In one embodiment, the suspension includes 2000 micrograms of iron oxide nanoparticles per milliliter of 50% glycerol. In certain embodiments, the suspension includes one or more enzymes selected from the group consisting of mutanase, dextranase and combinations thereof. In certain embodiments, the suspension includes 1% hydrogen peroxide and 1.75U/8.75U mutanase/dextranase.

In certain embodiments, the present disclosure provides a method for detecting and removing dental biofilm, wherein the method includes inserting the oral care device of the present disclosure into a mouth of the user, administering a suspension including iron oxide nanoparticles and hydrogen peroxide to the upper channel and the lower channel. One part of the iron oxide nanoparticles binds to the dental biofilm and another part of the iron oxide nanoparticles remains unbound. The method further includes administering 3,3',5,5'-tetramethylbenzidine to the upper channel and the lower channel, to stain the biofilm bound iron oxide nanoparticles blue; locating dental biofilm by detecting the bound iron oxide nanoparticles with a photodetector, actuating the unbound iron oxide nanoparticles with the one or more magnetic elements for assembly into antimicrobial robots or autonomous magnetic bristles or robots suitable for removal of dental biofilm, and mechanically removing the dental biofilm by applying the magnetic field to move the antimicrobial robots or autonomous magnetic bristles or robots along the surfaces having the bound iron oxide nanoparticles. In certain embodiments, the method further includes detecting an amount of the dental biofilm that is removed.

In certain embodiments, the method of the present disclosure further includes monitoring a level of plaque accumulation and a level of plaque removal, the method including collecting, at a server, data related to location and amount of dental biofilm, wherein the data results from detecting the bound iron oxide nanoparticles with the photodetector, transforming data into a numerical value that indicates the level of plaque accumulation and the level of plaque removal by a processor, and displaying a representation of the level of plaque accumulation and the level of plaque removal on a user interface of a terminal device or a web-based application. In certain embodiments, the representation of the level of plaque accumulation and the level of plaque removal is one or more of a numerical, a graphical or a color/visual output.

The accompanying drawings, which are incorporated and constitute part of this disclosure, illustrate preferred embodiments of the invention and serve to explain the principles of the invention.

### BRIEF DESCRIPTION OF THE DRAWINGS

FIG. 1A is a diagram illustrating the dual catalytic-magnetic functionality of the iron oxide nanoparticles.
FIG. 1B is a diagram illustrating biofilm resistance to antimicrobials and mechanical removal due to EPS matrix.
FIG. 1C is a diagram illustrating detection and disruption of biofilm.
FIG. 1D is a diagram illustrating magnetic actuation and removal of biofilm.
FIG. 2A depicts a device made of flexible material that adjust to the dental arch. Close-up shows the device having multifunctionality for removal of dental biofilm and biofilm detection and monitoring.
FIG. 2B depicts the device linked wirelessly to an app/mobile for monitoring oral health and hygiene (dental biofilm accumulation and removal).
FIG. 3A illustrates that the iron oxide nanoparticles do not bind to human gingival epithelial cells.
FIG. 3B illustrates that the iron oxide nanoparticles bind to biofilms.
FIG. 3C illustrates efficacy of biofilm bacteria eradication of iron oxide nanoparticles.
FIG. 4A depicts the catalytic activity of iron oxide nanoparticles in biofilm as a function of concentration of iron oxide nanoparticles. FIG. 4A inset further depicts the color change of the biofilm (to blue) after treatment of iron oxide nanoparticles, TMB and hydrogen peroxide.
FIG. 4B depicts the color change (to blue) of the biofilm on real teeth using mouth model after treatment of iron oxide nanoparticles, TMB and hydrogen peroxide. FIG. 4B further shows that TMB and hydrogen peroxide only stain biofilm, and do not stain teeth.
FIG. 4C shows a close up look of a tooth having stained biofilm without staining tooth surface.
FIG. 5A depicts plaque detection circuit used to detect blue color.
FIG. 5B shows that the indicator LED of the plaque detection circuit turns on when blue color is detected.
FIG. 5C shows that the indicator LED of the plaque detection circuit turns off when blue color is not detected.
FIG. 5D illustrate the relationship between the various amounts of biofilm removed and the blue color indicator used for biofilm (plaque) detection.
FIGS. 6A-6F illustrates iron oxide nanoparticles being manipulated back and forth by a magnetic element.
FIGS. 7A-D illustrate bristle-like structure the iron oxide nanoparticles assume on tooth surface in magnetic fields. FIG. 7B left panel shows magnetic field off, and Fig 7B middle and right panel show just after magnetic field being turned on, showing the assembly of the bristle-like structure.
FIGS. 8A-D illustrate the removal of plaque biofilm from enamel surface by magnetically-controlled movement of the bristle-like iron oxide nanoparticles robotic structure.
FIGS. 9A-C illustrate removal of plaque of biofilm from narrow interproximal areas.

### DETAILED DESCRIPTION

The presently disclosed subject matter provides oral care devices capable of eradicating the bacteria and degrading the biofilm matrix of dental plaque with a suspension including iron oxide nanoparticles. In certain embodiments, the devices of the present disclosure can also detect and monitor dental plaque. The devices of the present disclosure can further include sensors and micro processing properties, such as Bluetooth to allow for real-time monitoring of the amount of and removal of dental plaque. The present disclosure also provides for methods of eradicating the bacteria and degrading the biofilm matrix of dental plaque with suspensions including iron oxide nanoparticles that forms antimicrobial robots or autonomous magnetic bristles or robots.

For clarity and not by way of limitation, this detailed description is divided into the following sub-portions:
- Definitions;
- Iron Nanoparticle Suspensions;
- Oral Care Devices;
- Methods of Removing Dental Biofilms.

### Definitions

The terms used in this specification generally have their ordinary meanings in the art, within the context of this subject matter and in the specific context where each term is used. Certain terms are defined below to provide additional guidance in describing the compositions and methods of the disclosed subject matter and how to make and use them.

As used in the specification and the appended claims, the singular forms "a," "an" and "the" include plural referents unless the context clearly dictates otherwise. Thus, for example, reference to "a compound" includes mixtures of compounds.

In the detailed description herein, references to "embodiment," "an embodiment," "one embodiment", "in various embodiments," etc., indicate that the embodiment(s) described can include a particular feature, structure, or characteristic, but every embodiment might not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

The term "coupled" as used herein in reference to a photodetector and a light emitting diode refers to an electrical or wireless connection between the two components, wherein the light emitting diode can produce a visual indicator of the information detected by the photodetector.

Terms "dental biofilm" and "dental plaque" are used herein interchangeably and refer to a biofilm or mass of bacteria that grows on surfaces within the mouth.

As used herein, the term "user" includes mammals, such an animal or a human. In preferred embodiments, the subject is a human. In certain non-limiting embodiments, the "user" can be any individual using or interacting with the terminal device, which can use or interact with the user interface.

The term "terminal device" refers to, for example without limitation, a personal computer, laptop computer, workstation, mobile device, terminal device, or any other user equipment. In some non-limiting examples, the terminal device can include a graphical user interface used to display a graphical representation of, but not limited to, plaque detection, plaque removal and oral hygiene status to a user of the terminal device. In certain embodiments a web-based application can be installed on the user's terminal device. A user can monitor progress of removing dental plaque using the web-based application.

As used herein, "high frequency" refers to frequency of over 10 Hz.

### Iron Nanoparticle Suspensions

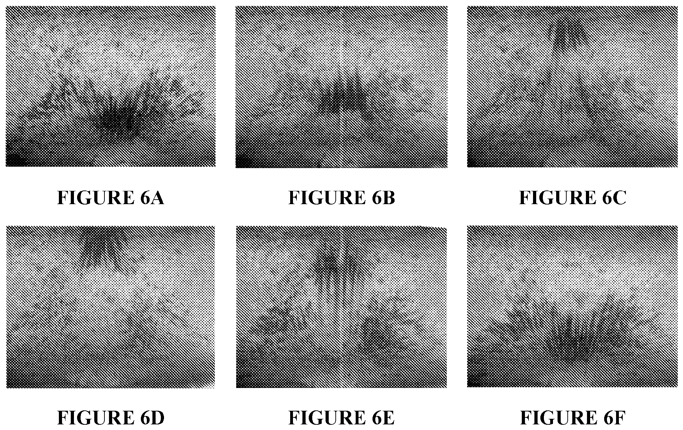

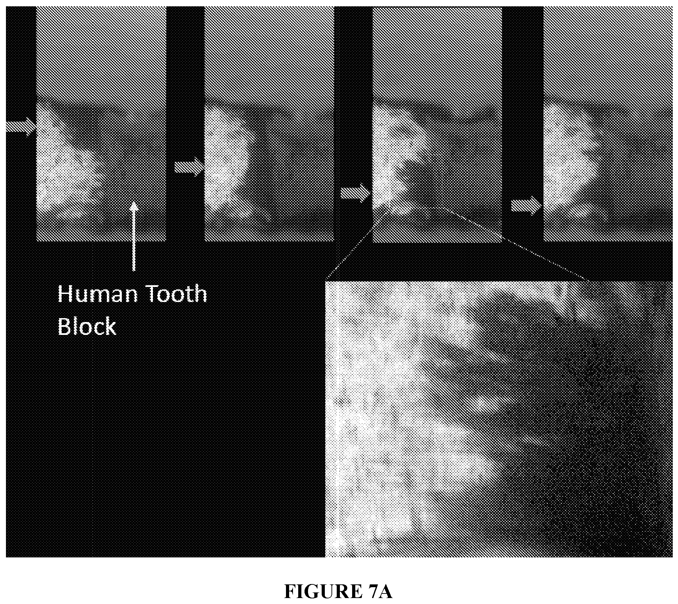

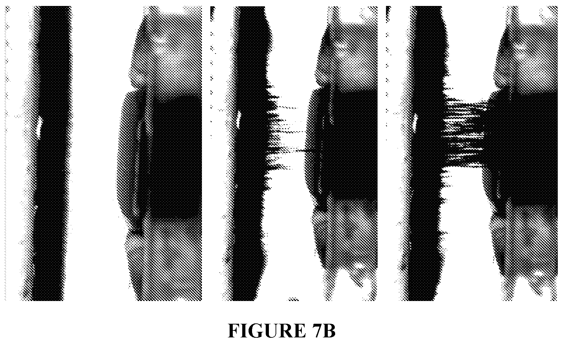

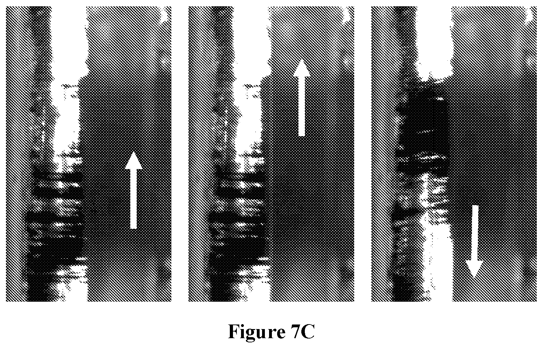

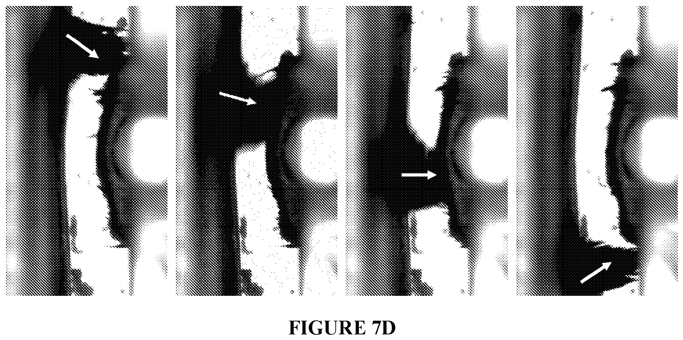

In certain embodiments, the iron oxide nanoparticles in accordance with the disclosed subject matter are capable of mechanically removing dental plaque. FIG. 6A illustrates that the iron oxide nanoparticles can form clusters in a magnetic field that are pulled along surfaces creating a controlled and swirled motion. FIGS. 7A-D illustrate a different mechanism where bristle-like constructs are formed on tooth surface in magnetic fields. As shown in FIGS. 7A and 7B, by controlling the magnetic field direction and gradient, bristle-like structures of iron oxide nanoparticles are formed from solution and extend to the enamel surface. FIG. 7C further shows that bristles of iron oxide nanoparticles can extend to the biofilm-covered surface and are actuated laterally to sweep and remove the biofilm. Notably, as shown in FIG. 7D, the bristles of iron oxide nanoparticles conform to the changing surface contours while degrading and removing biofilm.

In alternative embodiments, permanent magnets or electromagnets can be used while rotating, translating within the device and vibrating.

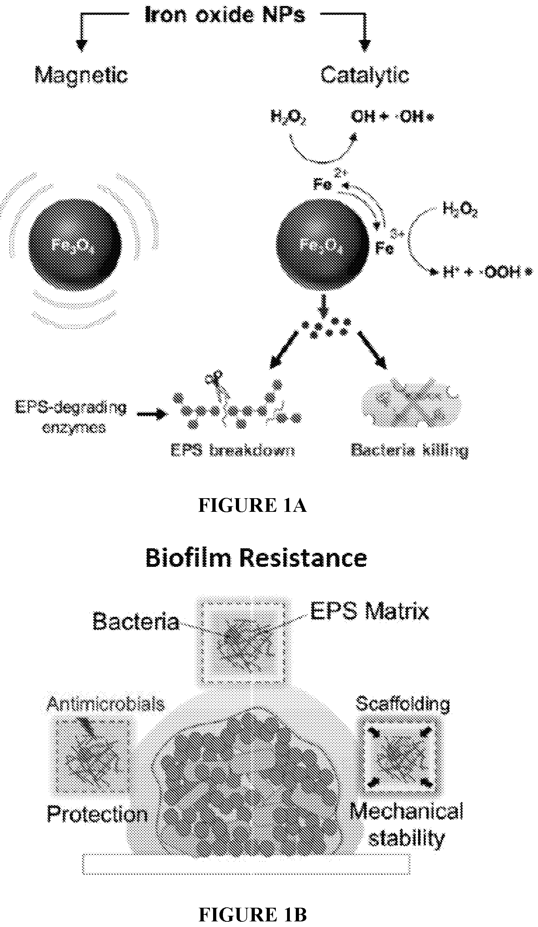

In certain embodiments, the suspension of the present disclosure includes hydrogen peroxide in addition to iron oxide nanoparticles. Iron oxide nanoparticles activate hydrogen peroxide to produce bioactive radicals capable of degrading dental biofilm and eradicating bacteria within dental biofilm. Particularly, iron oxide nanoparticles catalyze decomposition of hydrogen peroxide to produce free radicals, such as HO^{•} and HO₂^{•} radicals. FIGS. 1A and 1B illustrate the dual catalytic-magnetic functionality of the iron oxide nanoparticles in accordance with an embodiment of disclosed subject matter. The iron oxide nanoparticles can catalyze hydrogen peroxide (H₂O₂) to substantially eradicate the bacteria and degrade the biofilm matrix. The biofilm matrix degradation is key for disrupting the structural scaffold while also facilitating penetration and bacterial eradication. The bacterial eradication effect is substantially enhanced when the biofilm matrix is degraded. The biofilm matrix is degraded when it is sufficiently broken down to allow for substantial bacterial eradication (>99.999% killing). The iron oxide nanoparticles can be magnetically activated to actuate the iron oxide nanoparticles for assembly into antimicrobial robots or autonomous magnetic bristles or robots and to move the antimicrobial robots or autonomous magnetic bristles or robots to remove the biofilm debris.

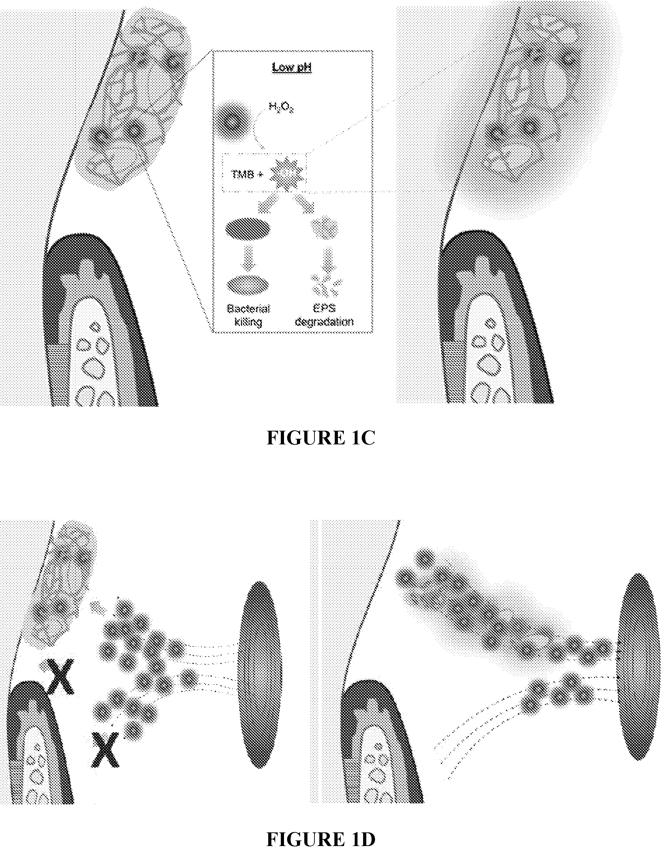

FIGS. 1C and 1D show the conceptual framework of the plaque removal and detection system using the catalytic antimicrobial robots. In Fig 1C the iron oxide nanoparticles bind and penetrates biofilms and catalyzes H2O2 in situ to breakdown the biofilm matrix and kill bacteria. At the same time, the nanoparticles can react with TMB during catalysis (see claim [0011]) to generate blue color that serves as indicator of plaque (biofilm), thereby serving as detector for biofilm accumulation. When the nanoparticles are actuated by the magnetic field (FIG. 1D left panel) it aggregates and assembles into bristle-like structures that remove and scrub-away the biofilm (FIG. 1D right panel); importantly, these nanoparticles do not bind to teeth or mucosal surface. Since these biofilms can be also labeled by blue color (see above), the amount of plaque removal can be also measured, thereby serving as plaque removal detection.

In certain embodiments, the suspension of the present disclosure includes a peroxidase sensitive dye in addition to iron oxide nanoparticles and hydrogen peroxide. In specific embodiments, the peroxidase sensitive dye is 3,3',5,5'-tetramethylbenzidine. Other dyes include: 2,2'-azino-bis(3-ethylbenzothiazoline-6-sulphonicacid (ABTS), o-phenylenediamine (OPD), 3, 3'-Diaminobenzidine (DAB), Pyrogallol, 4-amino-2,3-dimethyl-1-phenyl-3-pyrazolinone (4-Aminoantipyrine), 5-aminosalicylic acid (5-AS), 3-methyl-2-benzothiazolinone (MBTH) as well as fluorescent dyes including 10-Acetyl-3,7-dihydroxyphenoxazine, Terephthalic acid, Homovanillic acid, 2-[6-(4-aminophenoxy)-3-oxo-3H-xanthen-9-yl]-benzoic acid, (2-[6-(4'-hydroxy)phenoxy-3H-xanthene-3-on-9-yl]benzoic acid, 2',7'-Dichlorofluorescein diacetate, 2,7-Dichlorodihydrofluorescein diacetate, Coumarin Boronic Acid, Coumarin Boronic Acid pinacolate ester, Dihydrorhodamine 123, Lucigenin, Dihydroethidium. The iron oxide nanoparticles are capable of binding to dental biofilms and in the presence of hydrogen peroxide and a peroxidase sensitive dye generate a color that can be used to detect dental plaque.

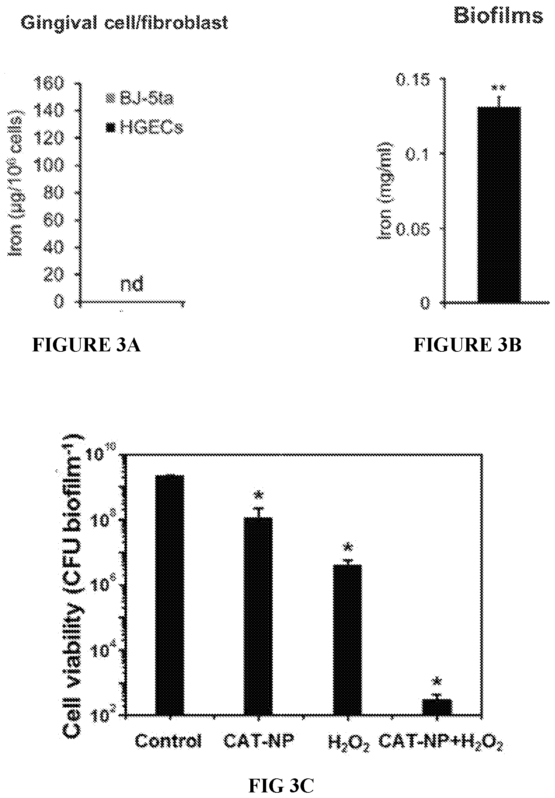

The suspension including iron oxide particles can further include one or more enzymes such as mutanase or dextranase to further help degrade the biofilm matrix. In certain embodiments, the suspension can be formulated with 1% hydrogen peroxide and 1.75U/8.75U mutanase/dextranase to substantially eradicate the bacteria and degrade the biofilm matrix. FIG. 3C illustrates efficacy of biofilm eradication of a suspension including iron oxide nanoparticles, hydrogen peroxide and mutanase/dextranase.

In certain embodiments, the suspension including iron oxide nanoparticles includes glycerol and/or water, or aqueous buffer. In certain embodiments, the suspension includes from about 500 micrograms to about 5000 micrograms, from about 750 micrograms to about 4750 micrograms, from about 1000 micrograms to about 4500 micrograms, from about 1250 micrograms to about 4250 micrograms, from about 1500 micrograms to about 4000 micrograms, from about 1750 micrograms to about 3750 micrograms, or from about 2000 micrograms to about 3500 micrograms of iron oxide nanoparticles per milliliter of 50% glycerol in water, or water, or aqueous buffer. In certain embodiments, the suspension includes less than 5000 micrograms, less than 4500 micrograms, less than 4000 micrograms, less than 3500 micrograms, less than 3000 micrograms, less than 2500 micrograms, less than 2000 micrograms, less than 1500 micrograms, or less than 1000 micrograms of iron oxide nanoparticles per milliliter of 50% glycerol in water, or water. In certain embodiments, the suspension includes at least 500 micrograms, at least 1000 micrograms, at least 1500 micrograms, at least 2000 micrograms, at least 2500 micrograms, at least 3000 micrograms, at least 3500 micrograms, at least 4000 micrograms, or at least 4500 micrograms of iron oxide nanoparticles per milliliter of 50% glycerol in water, or water. In one embodiment, the suspension includes 2000 micrograms of iron oxide nanoparticles per milliliter of 50% glycerol.

In certain embodiments, the suspension including iron oxide nanoparticles can include additional components commonly used in oral care compositions, such as but not limited to carbamide peroxide, antimicrobial compounds, fluoride ion sources, abrasive compounds, surfactants, detergents, enzymes, and combinations thereof. Non-limiting examples of antimicrobial compounds include triclosan, essential oils, terpenoids, flavonoids, polyphenols, proanthocyanidins, tannins, coumarins, chlorhexidine, antimicrobial peptides, arginine. Non-limiting examples of enzymes include dextranase, mutanase, lipases, DNAse, amyloglucosidade, glucose oxidase. Non-limiting examples of fluoride ion sources include alkali metal fluorides such as sodium fluoride, alkali metal monofluorophosphates, stannous fluoride and the like. Non-limiting examples of abrasive compounds include silica dental abrasives, calcium carbonate, dicalcium phosphate dihydrate, β-calcium pyrophosphate, insoluble alkali metal metaphosphates, plastic dental abrasives, and combinations thereof.

### Oral Care Devices

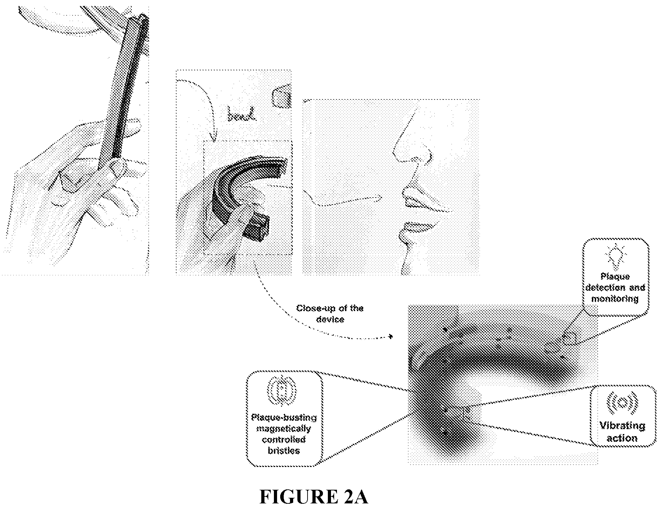

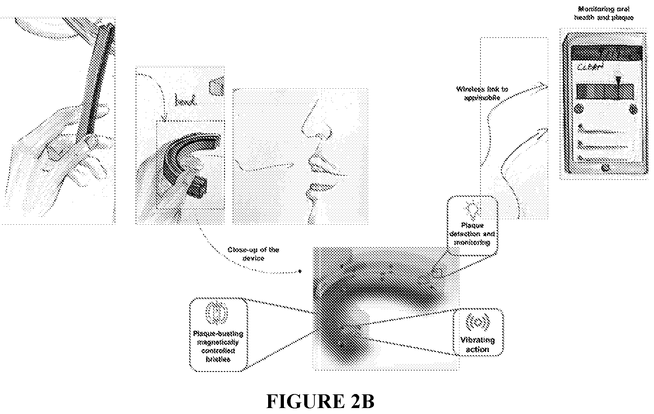

The devices of the present disclosure are configured to bring the suspension including iron oxide nanoparticles in contact with surfaces of teeth and gums of the user. In certain embodiments, the oral care device of the present disclosure includes a flexible mouthpiece including an upper channel configured to fit over upper teeth of the user and a lower channel configured to fit over lower teeth of the user. The mouthpiece can be sized to fit a range typical of humans from children to adults. The mouth opening for an adult ranges from 30-70 mm, and the total arch length for an adult ranges from 35-45 mm. In certain embodiments, the size of the mouthpiece is adjustable. FIGS. 2A and 2B provide a nonlimiting examples of the mouthpiece. Specifically, FIG. 2A shows an exemplary flexible mouthpiece, which includes an upper channel, a lower channel that can be adjusted to the dental arch. Close-up shows the device housing plaque-busting magnetically controlled bristles, and means for real-time plaque monitoring and means for providing a vibrating action.

FIG. 2B depict the device linked wirelessly to an app/mobile for monitoring oral health and hygiene (dental biofilm accumulation and removal).

In certain embodiments, the mouthpiece of the present disclosure includes one or more magnetic elements to generate a magnetic field. Nonlimiting examples of magnetic elements include electromagnets which include magnetic cores that are able to concentrate and amplify the pulling force. In certain embodiments, the magnetic gradients produced by these magnets can range from about 0.01 mT/millimeter to about 200 mT/millimeter, from about 1 mT/millimeter to about 180 mT/millimeter, from about 25 mT/millimeter to about 150 mT/millimeter, or from about 50 mT/millimeter to about 100 mT/millimeter. In certain embodiments, the magnetic gradients produced by these magnets are at least 1 mT/millimeter, at least 10 mT/millimeter, at least 25 mT/millimeter, at least 50 mT/millimeter, at least 75 mT/millimeter, at least 100 mT/millimeter, at least 125 mT/millimeter, at least 150 mT/millimeter, or at least 175 mT/millimeter. In certain embodiments, the maximum gradient does not exceed 200 mT/millimeter. Switching of the magnetic fields can occur across a range of frequencies including but not limited to values of from about 0.2 Hz to about 100 Hz, from about 5 Hz to about 75 Hz, form about 20 Hz to about 60 Hz, from about 30 Hz to about 50 Hz. In certain embodiments, the magnetic field is produced by permanent magnets such as but not limited to Iron, Iron Nickel alloys, Neodymium Iron Boron, and Samarium Cobalt.

In certain embodiments, the oral care device includes one or more haptic actuators (e.g. vibration actuators). In certain embodiments, the actuators used herein are small, oscillating devices (motors or piezoelectric materials) that produce vibration, can be felt (haptics), and can additionally resuspend nanoparticles and/or help remove biofilms. Nonlimiting examples of vibration actuators include rotating motors with unbalanced masses, which can be activated and varied in terms of frequency and amplitude of vibration. In certain embodiments, the one or more vibrating actuators are vibrating motors. A non-limiting commercial embodiment of a vibration actuator includes Z7AL2B1690002 by Jinlong Machinery & Electronics, Inc. In certain embodiments, the frequency of vibration varies from about 10 Hz to about 20,000 Hz. In certain nonlimiting embodiments, the vibrating action is induced by high frequency magnetic movements, wherein electromagnets and/or piezoelectric actuators transport iron oxide nanoparticles via high frequency oscillation of from about 10 Hz to about 20,000 Hz. In certain embodiments, the electromagnets responsible for moving iron oxide nanoparticles can also create mechanical vibration.

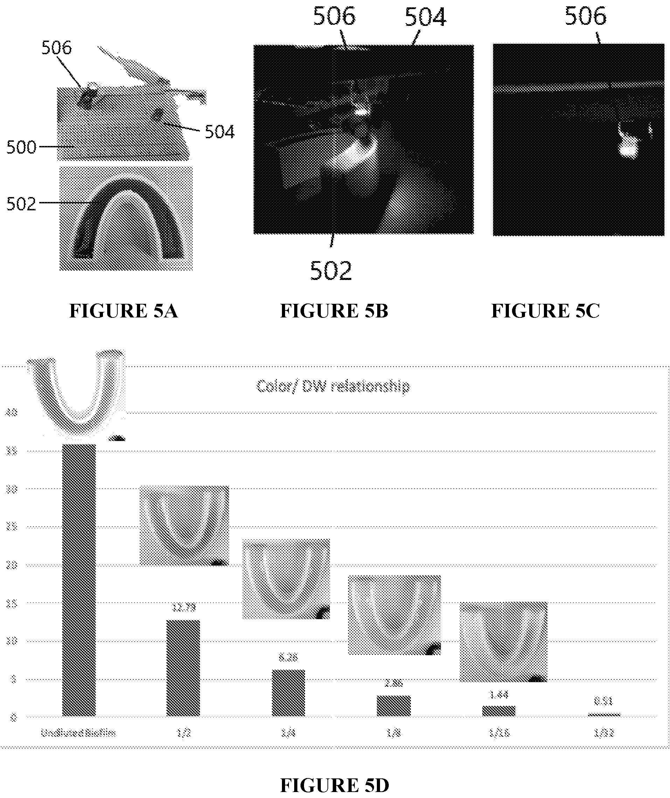

In certain embodiments, the oral care device of the present disclosure further includes a photodetector or RGB sensor. In certain embodiments, the photodetector or RGB sensor is coupled to a light-emitting diode to provide a visual indicator of a color or fluorescence detection. A nonlimiting example of such configuration is illustrated in FIG. 5A. FIG. 5A depicts an electric circuit 500 for detecting blue color in a tray 502. The circuit 500 includes a light-emitting diode 504 and a photodetector 506. FIGS. 5B and 5C illustrate that an indicator light-emitting diode 504 turns on when blue light is detected in the tray 502 by the photodetector 506 and remains off, when no blue color was detected.

The oral care device of the present disclosure is sized to enclose the battery and power electronics. In certain embodiments, the oral care device further includes micro processing capabilities.

In certain embodiments, the oral care device can be wirelessly connected to another device to monitor removal of dental biofilm in real-time.

### Methods of Detecting and Removing Dental Plaque

The present disclosure further relates to methods of removing dental plaque using oral care devices described herein. In certain embodiments, a user can remove dental plaque by administering the suspension disclosed herein including iron oxide nanoparticles to the upper channel and the lower channel of the mouthpiece and fitting the mouthpiece over the upper and lower teeth. Upon turning on the device, the iron oxide nanoparticles move due to the magnetic field and/or the vibrating action, forming antimicrobial robots or autonomous magnetic bristles or robots, thereby mechanically removing dental plaque.

In certain embodiments, the dental plaque can be removed by administering a suspension including iron oxide nanoparticles and hydrogen peroxide to the upper channel and the lower channel of the mouthpiece and fitting the mouthpiece over the upper and lower teeth. The iron oxide nanoparticles catalyze decomposition of hydrogen peroxide to produce HO^{•} and HO₂^{•} radicals which in turn eradicate bacteria within the biofilm and degrade biofilm. Furthermore, upon turning on the device, the iron oxide nanoparticles move due to the magnetic field and/or the vibrating action, thereby mechanically removing dental plaque.

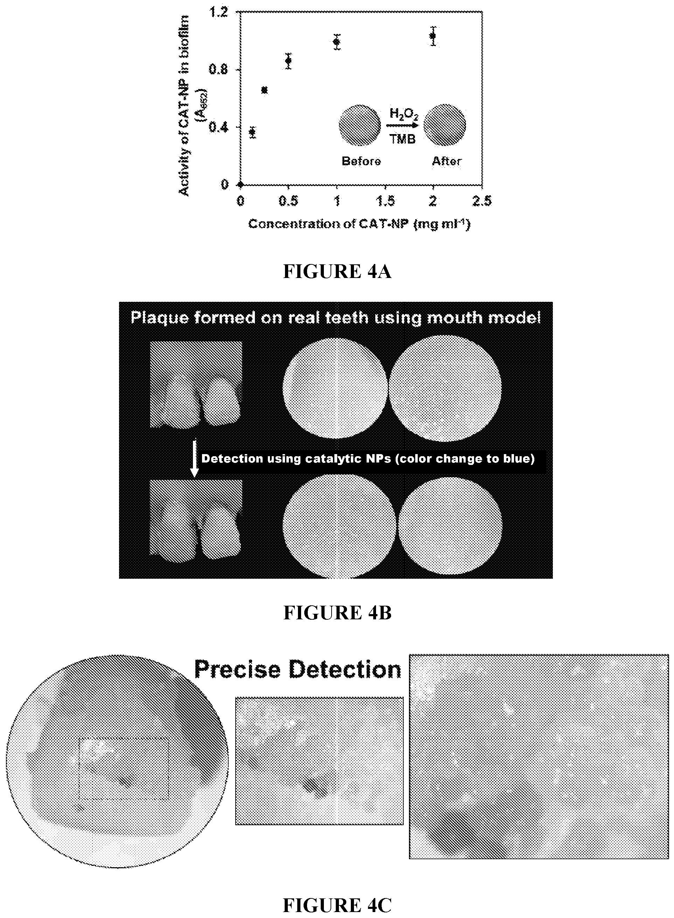

In certain embodiments, the dental plaque can be detected and removed by administering a suspension including iron oxide nanoparticles, hydrogen peroxide and a peroxidase sensitive dye to the upper channel and the lower channel of the mouthpiece and fitting the mouthpiece over the upper and lower teeth. A part of iron oxide nanoparticles binds to the dental plaque. The suspension further stains the plaque with bound iron oxide particles in blue, without staining teeth thereby serving as plaque detection, as illustrated in FIGS. 4A-4C. The visual indicator can be further used to identify location of dental plaque and subsequently direct the unbound iron oxide nanoparticles to the dental plaque towards the stained dental plaque to mechanically remove it. In addition, since the biofilm is stained in blue, the amount of plaque removal can be also measured, thereby serving as plaque (biofilm) removal detection.

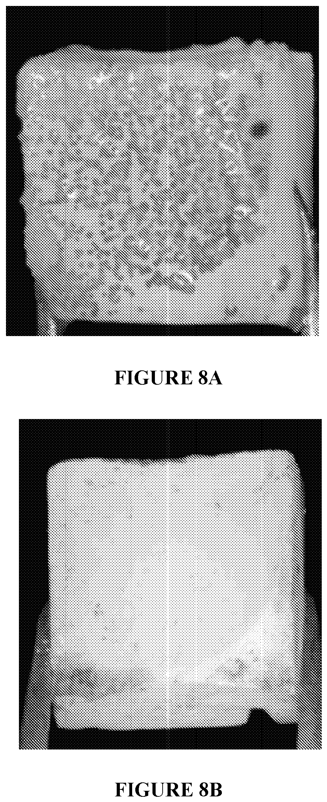

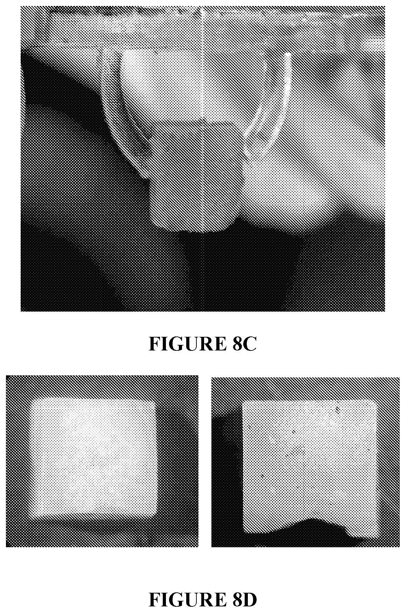

FIGS. 8A-D further illustrate the ability of antimicrobial robots or autonomous magnetic bristles or robots to remove dental plaque from teeth. FIGS. 8A and 8C show a "before" image of biofilm stained in pink formed on a tooth block and FIGS. 8B and 8D provide an "after image" that shows the same tooth block after it has been subjected to the automated cleaning by the autonomous magnetic bristles or robots, indicating complete biofilm removal.

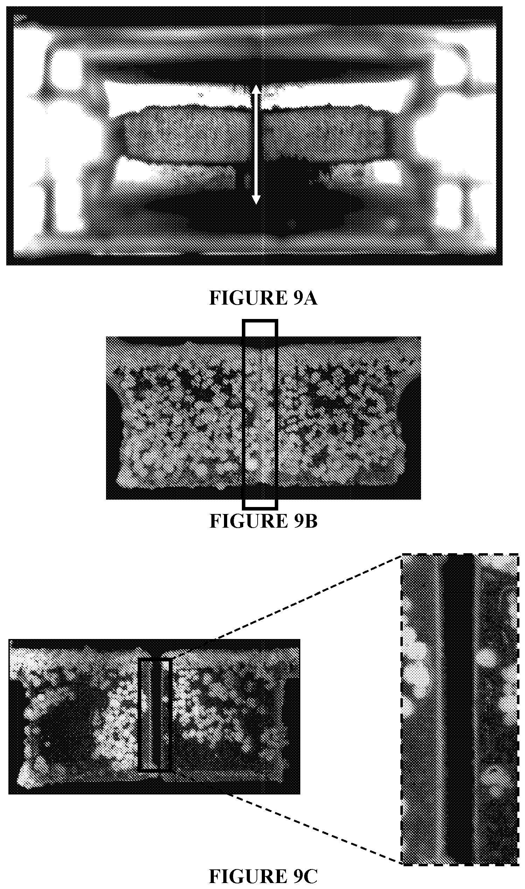

FIGS. 9A-C further illustrate the ability of antimicrobial robots or autonomous magnetic bristles or robots to remove dental plaque from narrow areas, such as, e.g., areas between the teeth. Such cleaning is performed by driving iron oxide nanoparticle assemblies through the interproximal space in a reciprocal motion. FIG. 9B provides "before" image showing plaque in a narrow area between two "model teeth" and FIG. 9C provides an "after" image showing that the plaque at the interproximal area has been removed; close-up view shows complete biofilm removal in the narrow space between the teeth.

In certain embodiments, level of plaque accumulation and level of plaque removal can be monitored and/or visualized on a terminal device or a web-based application in the terminal device. Once a photodetector or RGB sensor obtains data regarding dental plaque and amount thereof, the data can be transmitted for real-time monitoring and storage to a memory device located outside the sensor. The given sensor can transmit the obtained data to a server, which can then collect the data related to the amount of dental plaque and location thereof as well as plaque removal. In certain non-limiting embodiments, a centralized server can collect all of the data, while in other non-limiting embodiments the collection of data can be distributed to a plurality of servers. Each server can include one or more databases that store the collected information. When the centralized server collects all of the data, the data can be kept in one database or in multiple databases. The databases can include tags or identifiers describing characteristics of the collected data, such as the type of data collected. In certain embodiments, the data can be transformed into a numerical value that indicates level of plaque accumulation and level of plaque removal by a processor. In certain embodiments, the results can be visualized using a range of colors. In certain embodiments, the collected data can be used to monitor oral hygiene and oral health status over extended period of time. In certain embodiments, the web-based application or terminal device can be used to monitor real-time removal of dental plaque and real-time monitoring of amount of plaque accumulation on teeth.

The foregoing merely illustrates the principles of the disclosed subject matter. Various modifications and alterations to the described embodiments will be apparent to those skilled in the art in view of the teachings herein. It will thus be appreciated that those skilled in the art will be able to devise numerous techniques which, although not explicitly described herein, embody the principles of the disclosed subject matter.

## Claims

1. An oral care device comprising:
a mouthpiece having an upper channel configured to fit over upper teeth and a lower channel configured to fit over lower teeth of a user;
wherein the mouthpiece further comprises:
one or more magnetic elements, and
a vibrating motor,
wherein the mouthpiece contains a suspension comprising iron oxide nanoparticles, and
wherein the mouthpiece is configured to provide contact between the suspension and surfaces of upper teeth, lower teeth, including between the teeth, and gums of the user; wherein the one or more magnetic elements comprise a permanent magnet or electromagnet, adapted to apply a magnetic field to the biofilm to actuate the iron oxide nanoparticles to assemble into antimicrobial robots or autonomous magnetic bristles or robots, configurable to conform to changing surface contours,
**characterised in that**
the vibrating motor is configured to re-suspend the iron-oxide nanoparticles.

2. The device of claim 1, wherein the device further comprises a photodetector or RGB sensor.

3. The device of claim 2, wherein the photodetector is coupled to a light-emitting diode.

4. The device of claim 1, wherein the suspension further comprises a component selected from the group consisting of hydrogen peroxide, carbamide peroxide, one or more enzymes, one or more antimicrobial compounds, one or more surfactants, one or more detergents, one or more fluoride ion sources, one or more abrasive compounds, glycerol, one or more flavonoids, terpenoids, polyphenols, proanthocyanidins, tannins, coumarin, rose bengal, perborate, meta-periodate, sorbitol, xylitol, 1-deoxynojirimycin and combinations thereof.

5. The device of claim 4, wherein the suspension comprises hydrogen peroxide.

## Patentansprüche

1. Mundpflegevorrichtung, umfassend:
ein Mundstück mit einem oberen Kanal, der so konfiguriert ist, dass er über obere Zähne passt, und einem unteren Kanal, der so konfiguriert ist, dass er über untere Zähne eines Benutzers passt;
wobei das Mundstück ferner umfasst:
ein oder mehrere magnetische Elemente, und
einen Vibrationsmotor,
wobei das Mundstück eine Suspension enthält, die Eisenoxidnanopartikel umfasst, und
wobei das Mundstück so konfiguriert ist, dass es einen Kontakt zwischen der Suspension und Oberflächen der oberen Zähne, der unteren Zähne, einschließlich Zahnzwischenräumen, sowie dem Zahnfleisch des Benutzers bereitstellt; wobei das eine oder die mehreren magnetischen Elemente einen Permanentmagneten oder Elektromagneten umfassen, der dazu eingerichtet ist, ein Magnetfeld auf den Biofilm anzulegen, um die Eisenoxidnanopartikel zu aktivieren, sodass sie sich zu antimikrobiellen Robotern oder autonomen magnetischen Borsten oder Robotern zusammenfügen, die so konfigurierbar sind, dass sie sich verändernden Oberflächenkonturen anpassen, **dadurch gekennzeichnet, dass**
der Vibrationsmotor so konfiguriert ist, dass er die Eisenoxidnanopartikel resuspendiert.

2. Vorrichtung nach Anspruch 1, wobei die Vorrichtung ferner einen Photodetektor oder RGB-Sensor umfasst.

3. Vorrichtung nach Anspruch 2, wobei der Photodetektor an eine Leuchtdiode gekoppelt ist.

4. Vorrichtung nach Anspruch 1, wobei die Suspension ferner eine Komponente umfasst, die aus der Gruppe ausgewählt ist, bestehend aus Wasserstoffperoxid, Carbamidperoxid, einem oder mehreren Enzymen, einer oder mehreren antimikrobiellen Verbindungen, einem oder mehreren Tensiden, einem oder mehreren Reinigungsmitteln, einer oder mehreren Fluoridionenquellen, einer oder mehreren abrasiven Verbindungen, Glycerin, einem oder mehreren Flavonoiden, Terpenoiden, Polyphenolen, Proanthocyanidinen, Tanninen, Cumarin, Rose Bengal, Perborat, Meta-Periodat, Sorbit, Xylit, 1-Desoxynojirimycin sowie Kombinationen davon.

5. Vorrichtung nach Anspruch 4, wobei die Suspension Wasserstoffperoxid umfasst.

## Revendications

1. Dispositif de soins bucco-dentaires comprenant :
un embout buccal ayant un canal supérieur configuré pour s'adapter sur les dents supérieures et un canal inférieur configuré pour s'adapter sur les dents inférieures d'un utilisateur ;
dans lequel l'embout buccal comprend en outre :
un ou plusieurs éléments magnétiques, et
un moteur vibrant,
dans lequel l'embout buccal contient une suspension comprenant des nanoparticules d'oxyde de fer, et
dans lequel l'embout buccal est configuré pour assurer un contact entre la suspension et des surfaces de dents supérieures, de dents inférieures, y compris entre les dents, et de gencives de l'utilisateur ; dans lequel les un ou plusieurs éléments magnétiques comprennent un aimant permanent ou un électroaimant, adapté pour appliquer un champ magnétique au biofilm pour actionner les nanoparticules d'oxyde de fer afin qu'elles s'assemblent en robots antimicrobiens ou en poils magnétiques autonomes ou robots, configurables pour se conformer à des contours de surface changeants, **caractérisé en ce que**
le moteur vibrant est configuré pour remettre en suspension les nanoparticules d'oxyde de fer.

2. Dispositif selon la revendication 1, dans lequel le dispositif comprend en outre un photodétecteur ou un capteur RVB.

3. Dispositif selon la revendication 2, dans lequel le photodétecteur est couplé à une diode électroluminescente.

4. Dispositif selon la revendication 1, dans lequel la suspension comprend en outre un composant choisi dans le groupe constitué par le peroxyde d'hydrogène, le peroxyde de carbamide, une ou plusieurs enzymes, un ou plusieurs composés antimicrobiens, un ou plusieurs tensioactifs, un ou plusieurs détergents, une ou plusieurs sources d'ions fluorure, un ou plusieurs composés abrasifs, le glycérol, un ou plusieurs flavonoïdes, terpénoïdes, polyphénols, proanthocyanidines, tannins, la coumarine, le rose bengale, le perborate, le méta-périodate, le sorbitol, le xylitol, la 1-désoxynojirimycine et des combinaisons de ceux-ci.

5. Dispositif selon la revendication 4, dans lequel la suspension comprend du peroxyde d'hydrogène.
